# EUROPEAN PATENT APPLICATION

(11) **EP 0 941 736 A1**
(43) Date of publication of application: **15.09.1999**
(21) Application number: 99104209.4
(22) Date of filing: 02.03.1999
(51) Int. Cl.: A61K 35/66

(54) **Oral immunity enhancing agent containing peptidoglycans**

(30) Priority: 10.03.1998 JP 5793698
(71) Applicant: Ajinomoto Co., Ltd., Tokyo (JP)
(72) Inventor: Yamazaki, Masatoshi, Hachioji-shi, Tokyo (JP); Sakuda, Shohei, Chiba-shi, Chiba-ken (JP); Toride, Yasuhiko c/o Ajinomoto Co., Inc., Tokyo (JP)
(74) Representative: Strehl Schübel-Hopf & Partner

(57) **Abstract**

An oral immunity enhancing agent contains peptidoglycans (PG) substantially free from muramyl dipeptide and having a molecular weight of not higher than 10,000 as an active ingredient, which makes it possible to enhance the immunity of mammals by the oral administration.

## Description

### Background of the Invention

The present invention relates to an oral immunity enhancing agent capable of enhancing the immunity of mammals (preferably animals excluding human beings), fishes and crustaceans.

It has been already well-known that peptidoglycans which are main constituents of cell walls constituting microorganisms have an immunopotentiating property in animals widely ranging from the lower animals to the higher animals. Various patent applications were filed in the field of aquaculture of marine products. For example, Japanese Patent Publication for Opposition Purpose (hereinafter referred to as "J. P. KOKOKU") No. Hei 6-25067 discloses a method of preventing infection of diarrhea of weanling piglets, characterized by repeatedly administering peptidoglycans (muramyl dipeptide polymers) to three-week-old piglets.

However, it was pointed out that when the muramyl dipeptide polymers are practically given to fishes or the like by the oral administration, the appearance of the immunopotentiating activity thereof is not clear. Among them, N-acetylmuramyl-L-alanyl-D-isoglutamine (muramyl dipeptide, MDP: molecular weight: 492) which belongs to the muramyl dipeptide family and which is known to have the immunopotentiating activity is expensive and the use thereof in the stock-raising industry is unexpectable, although it has a priming effect for activating macrophages through oral administration.

### Summary of the Invention

An object of the present invention is to provide an immunity enhancing agent capable of enhancing the immunity of mammals by the oral administration.

This and other objects of the present invention will be apparent from the following description and Examples.

The present invention has been completed on the basis of findings that the biological activity varies depending on a difference in the molecular weight of molecules constituting the peptidoglycans, i.e. muramyl dipeptide family or, in other words, the peptidoglycans having a molecular weight of not higher than 10,000, obtained by treating the cell walls of microorganisms with an enzyme such as lysozyme or protease, exhibit an excellent immunity enhancing effect when it is orally administered.

Namely, the present invention provides an oral immunity enhancing agent containing a peptidoglycan (PG) substantially free from muramyl dipeptide and having a molecular weight of not higher than 10,000 as an active ingredient.

### Preferred Embodiments of the Invention

The peptidoglycans (PG) substantially free from muramyl dipeptide and having a molecular weight of not higher than 10,000 used in the present invention can be obtained by treating the cell walls of microorganisms of the genus Bacillus, Brevibacterium, Corynebacterium, Escherichia, Lactobacillus, Streptococcus, Streptomyces or Bifidobacterium with an enzyme such as lysozyme or protease to remove fractions having a molecular weight of larger than 10,000.

In the present invention, there can be employed microorganisms, culture methods and method of disintegrating the cell walls described from line 34, column 3 to line 37, column 4 of Japanese Patent No. 2,526,733, the disclosure of which is incorporated herein by reference.

In the above-described microorganisms, those of the genus Bacillus, Brevibacterium and Corynebacterium are preferably used. The peptidoglycans contained in cell walls of microorganisms have the general structure of a saccharide peptide polymer comprising repeating units of two saccharides, N-acetylglucosamine (GlcNAc) and N-acetylmuramic acid (MurNAc), bound via a β-1,4-glycoside bond, to which repeating unit a peptide chain comprising four amino acids is bonded. When (albumen) lysozyme is used as the enzyme for the treatment, it decomposes the bonds of MurNAc and GlcNAc but it cannot cut the bond between MurNAc and GlcNAc and, therefore, the minimum unit of the saccharides obtained by this treatment is the disaccharide. M-acetylmuramyl-L-alanyl-D-isoglutamine (muramyl dipeptide, MDP; molecular weight: 492) generally known to be the minimum effective structure of peptidoglycan having the immunopotentiating activity is not formed. Thus lysozyme is preferably used for the enzymatic treatment.

The fraction of a molecular weight of not higher than 10,000 in the cell walls of the microorganisms treated with the enzyme may be purified by a known method of molecular weight fractionation of proteins or the like or, alternatively, the product containing the fraction may be used as it is after the treatment with the enzyme without purification.

Although the oral immunity enhancing agent of the present invention preferably contains only peptidoglycans (PG) having a molecular weight of not higher than 10,000 and substantially free of muramyl dipeptide, it may contain a peptidoglycans having a molecular weight of higher than 10,000. However, when the peptidoglycans of a molecular weight of higher than 10,000 are contained therein in a large amount, a sufficient immunopotentiating effect cannot be obtained even the dose thereof is increased. Therefore, the amount of the peptidoglycans having a molecular weight of not higher than 10,000 is preferably at least 10 % by weight (hereinafter referred to as "%"), more preferably at least 40 %, based on the whole peptidoglycans. As will be shown in Examples given below, it is surprising that an excellent immunopotentiating effect can be obtained by giving even only 1 mg of peptidoglycans containing about 15 % of peptidoglycans having a molecular weight of not higher than 10,000 to mice, while the immunopotentiating effect cannot be obtained by giving even 20 mg of peptidoglycans containing about 1 % of the peptidoglycans having a molecular weight of not higher than 10,000.

The molecular weight of the peptidoglycans used in the present invention is not higher than 10,000, preferably 500 to 10,000 and more preferably 500 to 4,000. The molecular weight can be determined by the gel filtration method, light scattering method or the like.

The dose of the peptidoglycan having a molecular weight of not higher than 10,000 and substantially free of MDP which is well-known to have the immunopotentiating effect is orally administered preferably at least 0.5 mg/kg-body weight, more preferably at least 1 mg/kg-body weight in the present invention. The animals to which the peptidoglycans are to be administered include mammals (preferably animals excluding human beings), fishes and crustaceans.

The immunity enhancing agent of the present invention can be directly and orally given to the animals or added to a food which is to be given to the animals.

The time of the administration is not particularly limited. When the immunity enhancing agent is to be administered to fishes and crustaceans, the preventive effect thereof can be obtained when the administration is started in the fry period.

When the immunity enhancing agent of the present invention is to be added to the food, it is incorporated into starting materials for the food usually used for raising domestic animals or for aquaculture of fishes, and the amount thereof is suitably determined depending on the animals or fishes.

According to the present invention, there can be provided an immunity enhancing agent capable of enhancing the immunity of mammals by the oral administration without necessitating expensive MDP.

The following Examples will further illustrate the present invention.

### Example 1

### (1-1) Method of culturing microbial cells and method of obtaining the cells:

A liquid culture medium having a composition shown below was sterilized in an autoclave (121 °C, 15 minutes) and then inoculated with Brevibacterium lactofermentum ATCC 13869. After the shaking culture at 30 °C for 24 hours, the culture liquid was centrifuged at 7,000 rpm for 10 minutes to remove the culture medium. The microbial cells thus collected were suspended in 0.9 % aqueous NaCl solution, and the obtained suspension was centrifuged at 7,000 rpm for 10 minutes and washed to obtain the cleaned cells.
1.0 g/cc Glucose
1.0 g/cc Yeast extract
1.0g/cc Peptone
0.5 g/cc (NH₄)₂SO₄
0.3 g/cc K₂HPO₄
0.1 g/cc KH₂PO₄
0.05 g/cc MgSO₄, and
The balance: Distilled water
pH: 7 (adjusted with KOH).

### (1-2) Enzymatic treatment of cell wall components:

The microbial cells obtained by the above-described method (1-1) were suspended in distilled water, and the obtained suspension was treated with ultrasonic waves (ultrasonic disintegration machine: UR-200P, a product of TOMY SEIKO CO., LTD. frequency: 20 kHz, 200 W, 30 minutes). 3,000 g of the suspension was centrifuged for 30 minutes to remove the cells remaining not disintegrated. Then the supernatant liquid was centrifuged at 18,000 rpm for 30 minutes. The obtained precipitate was suspended in 4 % aqueous SDS solution, and the obtained suspension was heat-treated at 100°C for 40 minutes. After cooling to room temperature, the centrifugation was conducted again at 18,000 rpm at 25 °C for 30 minutes.
The centrifugation was repeated again, and the precipitate thus obtained was freeze-dried and treated with 0.01 % of albumen lysozyme (a product of Sigma Co.) and 0.01 % of achromopeptidase (also a product of Sigma Co.) for 72 hours. The supernatant liquid obtained by the centrifugation was recovered to obtain the soluble fraction of cell walls mainly comprising the peptidoglycans.

### (2-1) Separation of fraction having a molecular weight of not higher than 10,000:

The soluble fraction of cell walls obtained in (1-2) was dissolved in a suitable amount of distilled water, and the obtained solution was passed through a polysulfone ultrafilter capable of passing materials having a molecular weight of not more than 10,000 to conduct the fractionation.

### (2-2) Evaluation of TNF- α induction effect by oral administration to mice:

Each of the soluble fraction of cell walls having a molecular weight of higher than 10,000 and that not higher than 10,000 both obtained in (1-2) was orally administered to ICR mice (average body weight: 20 g). Three hours after, a commercially available immunopotentiating agent (OK-432; a product of Chugai Pharmaceutical Co., Ltd.) was intraveneously administered and, two hours after, TNF-α activity in the serum was determined. Distilled water was used as the control sample, and muramyl dipeptide (Sigma; MDP) was used as the standard for the determination of the immunopotentiating effect.

The quantities of the peptidoglycans contained in the fraction of cell walls having a molecular weight of higher than 10,000 and that not higher than 10,000 were made equal by determining diaminopiperic acid, which is an amino acid peculiar to the peptidoglycans, as the index, and the priming effect on TNF production in the blood in each case was evaluated by the method shown in Table 1. The results are shown in Table 2.

**Table 1**

| (Sample and dose) | |
|---|---|
| Evaluated sample | Dose |
| Distilled water | 1.0 mg/mouse |
| Fraction of mol. wt. of higher than 10,000 | 1.0 mg/mouse |
| Fraction of mol. wt. of higher than 10,000 | 2.0 mg/mouse |
| Fraction of mol. wt. of not higher than 10,000 | 0.5 mg/mouse |
| Fraction of mol. wt. of not higher than 10,000 | 1.0 mg/mouse |
| MDP | 0.1 mg/mouse |

**Table 2**

| (Results) | |
|---|---|
| | Priming in TNF |
| production | |
| Distilled water (1.0 mg/mouse) | - |
| Fraction of mol. wt. of higher than 10,000 (1.0 mg/mouse) | - |
| Fraction of mol. wt. of higher than 10,000 (2.0 mg/mouse) | - |
| Fraction of mol. wt. of not higher than 10,000 (0.5 mg/mouse) | + |
| Fraction of mol. wt. of not higher than 10,000 (1.0 mg/mouse) | ++ |
| MDP (0.1 mg/mouse) | ++ |

The priming effect in the TNF production in the blood was determined by the relative evaluation wherein the effect obtained by the oral administration of 0.1 mg/mouse of MDP was shown as "++".

It is apparent from the results given in Table 2 that the fraction of a molecular weight of not higher than 10,000 had a priming effect equivalent to that of MDP in the TNF production and that it exhibited an excellent immunopotentiating effect after the oral administration. However, the fraction of a molecular weight of higher than 10,000 had no priming effect in the TNF production.

In this Example, the albumen lysozyme was used for the enzyme for the treatment. Since the lysozyme decomposes the bonds of MurNAc and GlcNAc but it cannot cut the bond between MurNAc and GlcNAc, the minimum unit of the saccharides obtained by this treatment was the disaccharide, and MDP generally known to be the minimum effective structure of the peptidoglycans having the immunopotentiating effect was not formed. Therefore, it is apparent that the immunopotentiating effect obtained in this test is not of MDP.

### Example 2

### Investigations on the optimum dose of microbial cells treated with enzyme and containing low-molecular fraction having immunopotentiating activity:

The microbial cells of Bacillus subtilis (ATCC 13952) and Brevibacterium lactofermentum (ATCC 13869) were prepared in the same manner as that in method (1-1) of Example 1.

The microbial cells thus obtained were suspended in distilled water, and the obtained suspension was treated with ultrasonic waves (ultrasonic disintegration machine: UR-200P, a product of TOMY SEIKO CO., LTD.; frequency: 20 kHz, 200 W, 30 minutes). The precipitate obtained by the centrifugation of the suspension was recovered and the cells remaining not disintegrated were removed. Then the supernatant liquid was centrifuged at 18,000 prm for 30 minutes. The obtained precipitate was suspended in 4 % aqueous SDS solution, and the obtained suspension was heat-treated at 100°C for 40 minutes. After cooling to room temperature, the centrifugation was conducted again at 18,000 rpm at 25 °C for 30 minutes. The centrifugation was repeated again, and the precipitate thus obtained was freeze-dried and treated with 0.01 % of lysozyme and 0.01 % of achromopeptidase. The reaction periods for Brevibacterium lactofermentum were 8 hours and 72 hours, and that for Bacillus subtilis was 48 hours. After the completion of the enzymatic treatment, the supernatant liquid obtained by the centrifugation was recovered to obtain the soluble fraction of cell walls.

To determine the optimum dose for exhibiting the immunopotentiating activity, 0.5, 1, 5, 10 and 20 mg/mouse of each sample was given to ICR mice (average body weight: 20.0 g), and TNF-α induction effects were compared with one another by method (2-2) in Example 1.

The quantities of the peptidoglycans contained in the soluble fraction of cell walls obtained under the various conditions were determined by determining diaminopimeric acid, which is an amino acid peculiar to the peptidoglycans, as the index. The isolation and identification were conducted by the gel filtration to find the relative amounts of the fraction having a molecular weight of not higher than 10,000 in the peptidoglycans. The results are shown in Table 3.

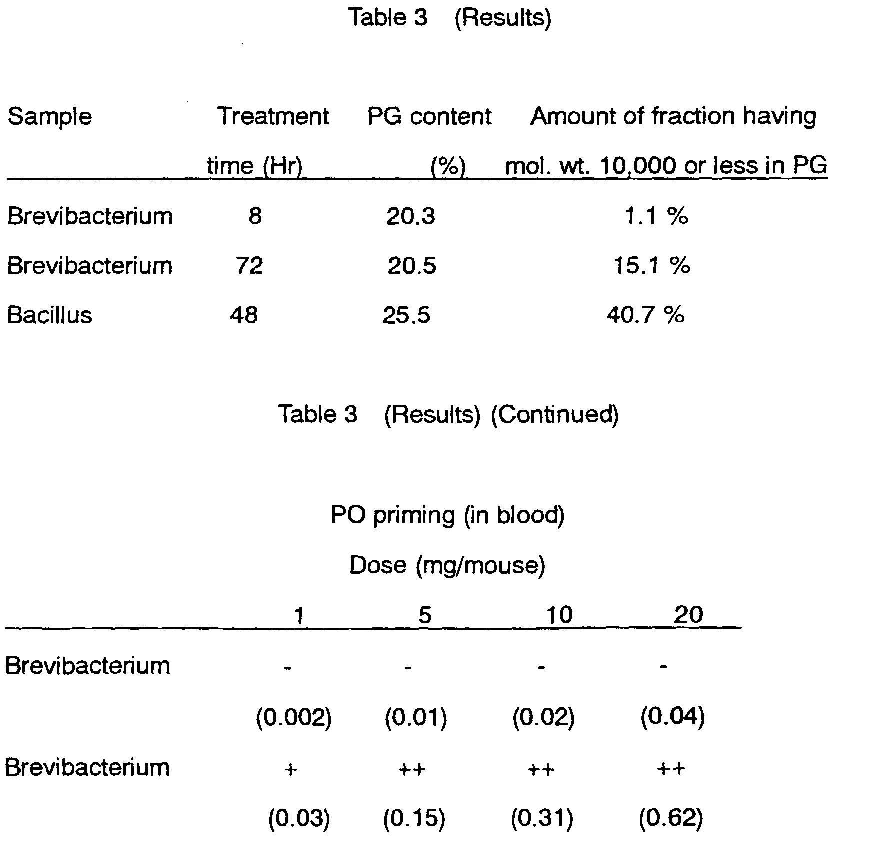

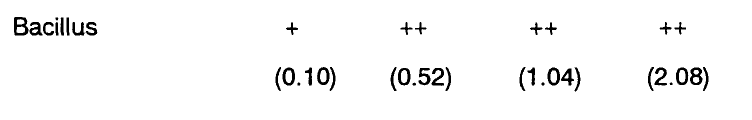

In Table 3, the numerals in parentheses indicate the dose/mouse of the fraction of a molecular weight of not higher than 10,000.

The priming effect in TNF production in the blood was evaluated relatively to the effect (++) obtained by the oral administration of 0-1 mg/mouse of MDP.

As shown in Table 3, the relative amount of the fraction having a molecular weight of not higher than 10,000 and having the immunopotentiating property in the sample derived from Brevibacterium lactofermentum was increased from 1.1 % to 15.1 % by changing the enzymatic treatment time from 8 hours to 72 hours. When Bacilus subtilis was treated with the enzyme for 48 hours, the relative amount of the fraction having a molecular weight of not higher than 10,000 in the peptidoglycans was 40.7 %.

### Example 3

### Investigations on the immunopotentiating activity of low molecular fraction of microbial cell walls treated with enzyme in the oral administration to shrimps (black tigers):

Brevibacterium lactofermentum (ATCC 13869) was cultured in the same manner as that of Example 1. Then the microbial cell wall components were treated with the enzyme. The enzymatically treated microbial cells were treated with the enzyme in the same manner as that of Example 2 for 8 hours or 72 hours to obtain the respective soluble fractions of the cell walls.

0 %, 0.005 %, 0.01 % and 0.1 % of the sample obtained by the 8 hours treatment and supposed to be rich in the fraction having a molecular weight of higher than 10,000 and the sample obtained by the 72 hours treatment and supposed to be rich in the fraction having a molecular weight of not higher than 10,000 were added to a basic feed having the following composition to obtain the test feeds.

| Basic feed: | |
|---|---|
| Material | Relative amount (%) |
| Fish grounds | 28 |
| Shrimp powder | 10 |
| Squid meal | 2 |
| Squid liver powder | 3 |
| Wheat gluten | 6 |
| Wheat flour | 20 |
| Soybean cake | 10 |
| Rice bran | 10.77 |
| Fish oil | 2 |
| Lecithin | 2 |
| Zeolite | 1.5 |
| Cholesterol | 0.5 |
| Vitamins | 0.88 |
| Minerals | 4.02 |
| Cellulose | 0.02 |

20 shrimps weighing 0.70 to 0.75 g were raised in each of 200 l water tanks provided with an aerating means and refluxing means. In the course of the raising, the sea water was kept at 6.0 to 7.0 ppm DO, 28 to 30 ppt salinity and 0.001 ppm NH₃ concentration. The mixed feed shown in the above Table was given four times a day to raise the shrimps for eight weeks. Eight weeks after, blood was taken and the phagocytosis of the hemocyte fraction (mainly macrophages) was determined, and the results of the respective test groups are compared with one another.

The blood thus obtained was taken by means of a l ml plastic syringe designed so that it would be mixed with an anticoagulant [0.45 mg cysteine / Lobster haemolymph medium (LHM); by a method of Paterson and Stewart (1974)] at a volume ratio of 1:1. After thorough stirring, the mixture was centrifuged at 1,000 rpm at 4°C for 10 minutes. LHM was added again to the obtained hemocyte fraction, and the obtained mixture was stirred and then centrifuged at 1,000 rpm at 4°C for 10 minutes. This process was repeated three times in total. LHM medium having a cell number in the blood fraction controlled at a concentration of 1 × 10⁶ cells/ml was mixed with an equal amount of LHM medium containing latex beads controlled at 1 × 10⁶ beads/ml. The mixture was placed on a slide glass and cultured in a humidified chamber for 45 minutes. After the completion of the culture, the cells on the slide glass were fixed with an LHM solution containing 0.125 % of glutaraldehyde and subjected to the giemsa staining. Immediately after the staining, the numbers of the total cells and the cells which had phagocytized the beads were counted with a phase contrast microscope. The phagocytizing capacities were compared with each other in terms of the ratio of the number of the cells which phagocytized the beads to the number of the total cells. The quantities of the peptidoglycans contained in the soluble fraction of cell walls obtained under the various conditions were determined by determining diaminopiperic acid, which is an amino acid peculiar to the peptidoglycans, as the index. The isolation and identification were conducted by the gel filtration to find the relative amounts of the fraction having a molecular weight of not higher than 10,000 in the peptidoglycans.

The results are shown in Table 4.

**Table 4**

| (Comparison of phagocytosis) | | | | |
|---|---|---|---|---|
| | Addition rate (%) | | | |
| | 0 | 0.005 | 0.01 | 0.1 |
| After treatment for 8 hours | 16.9 (0) | 30.9 (0.11) | 39.4 (0.22) | 28.0 (2.2) |
| After treatment for 72 hours | 16.5 (0) | 53.5 (1.55) | 68.3 (3.1) | 70.7 (31.0) |
| Numeral in the parentheses: the amount (g) of the fraction having a molecular weight of not higher than 10,000 in PG per ton of the feed. | | | | |

The peptidoglycan contents of both samples (sample treated for 8 hours and that treated for 72 hours) were substantially equal to each other. The enzymatic treatment time is different (8 hours and 72 hours), and the amount of the fraction having a molecular weight of not higher than 10,000 and contained in the peptidoglycans was 1.1 % after the treatment for 8 hours and 15.1 % after the treatment for 72 hours (see Table 3).

The results obtained by using the above-described samples were as follows: As shown in Table 4, the phagocytic capacity of the hemocytes was increased as the addition rate was increased in the samples treated for 72 hours, and it is apparent from the comparison of the samples of the equal addition rate that the phagocytic capacity of them was higher than that of the samples treated for 8 hours. On the other hand, in the samples treated for 8 hours, the phagocytic capacity depended on the volume till the addition rate of 0.01 % and, after that, the phagocytic capacity was no more increased by increasing the addition rate from 0.01 % to 0.1 %. The highest phagocytic capacity was obtained at the addition rate of 0.01 %.

It is understood from the above-described results of the comparison of the phagocytic capacities obtained by the oral administration that the activity of the samples treated for 72 hours and having a larger amount of the fraction of a molecular weight of not higher than 10,000 was higher than that of the samples treated for 8 hours and having a larger amount of the fraction of a molecular weight of higher than 10,000. It was also shown that the effect of the samples treated for 72 hours varies depending on the dose, while that of the samples treated for 8 hours was not improved and rather reduced even though the dose is increased to 0.01 % or more.

## Claims

1. An oral immunity enhancing agent containing peptidoglycans (PG) substantially free from muramyl dipeptide and having a molecular weight of not higher than 10,000 as an active ingredient.

2. The immunity enhancing agent of claim 1 which contains at least 10 % by weight of the peptidoglycans.

3. The immunity enhancing agent of claim 1 which contains at least 40 % by weight of the peptidoglycans.

4. The immunity enhancing agent of claim 1 which essentially consists of the peptidoglycans.

5. The immunity enhancing agent of claim 1 wherein the molecular weight is 500 to 10,000.

6. The immunity enhancing agent of claim 1 wherein the molecular weight is 500 to 4,000.

7. The immunity enhancing agent of claim 1 wherein the peptidoglycans are those obtained from the microorganisms selected from the group consisting of genus Bacillus, Brevibacterium and Corynebacterium.

8. An oral immunity enhancing agent containing at least 40 % by weight of peptidoglycans (PG) substantially free from muramyl dipeptide and having a molecular weight of 500 to 10,000 as an active ingredient.

9. The immunity enhancing agent of claim 8 which essentially consists of the peptidoglycans.

10. The use of peptidoglycans (PG) substantially free from muramyl dipeptide and as characterised in any of the claims 1 to 9 for the preparation of an immunity enhancing agent containing the peptidoglycans or essentially consisting of the peptidoglycans.
